# EUROPEAN PATENT SPECIFICATION

(11) **EP 1 838 445 B1**
(45) Date of publication and mention of the grant of the patent: **06.11.2013**
(21) Application number: 05787900.9
(22) Date of filing: 21.09.2005
(51) Int. Cl.: G01N 1/42, A61D 19/02, B01L 3/00, C12M 1/26, C12M 1/00, C12N 1/04, A01N 1/02, C12N 5/02, C12M 3/00

(54) **RETRACTABLE TOOL AND METHOD FOR MANIPULATING DEVELOPMENTAL CELLS IN A PROCESS OF CRYOPRESERVATION**
EINZIEHBARES WERKZEUG UND VERFAHREN ZUR HANDHABUNG VON ENTWICKLUNGSZELLEN IN EINEM TIEFKÜHLKONSERVIERUNGSVERFAHREN
OUTIL RETRACTABLE ET PROCEDE DE MANIPULATION DE CELLULES DU DEVELOPPEMENT LORS DE LEUR CRYOCONSERVATION

(30) Priority: 21.12.2004 US 637466 P
(43) Date of publication of application: 03.10.2007
(73) Proprietor: McGill University, Montreal, QC H3A 2T5 (CA)
(72) Inventor: TAN, Seang Lin, Westmount, Québec H3Y 1P3 (CA); CHIAN, Ri-Cheng, Longueuil, Québec J4M 2Y6 (CA)
(74) Representative: Plougmann & Vingtoft A/S
(86) International application number: PCT/CA2005/001441
(87) International publication number: WO 2006/066385

(56) References cited:
- EP-B1- 1 121 015
- WO-A1-02/085110
- WO-A1-2004/092358
- FR-A1- 2 574 919
- US-A- 5 545 562
- ISACHENKO V ET AL: "Aseptic technology of vitrification of human pronuclear oocytes using open-pulled straws", February 2005 (2005-02), HUMAN REPRODUCTION (OXFORD), VOL. 20, NR. 2, PAGE(S) 492-496, XP009150823, ISSN: 0268-1161 * figure 1 *
- VANDERZWALMEN P ET AL: "Vitrification of human blastocysts with the Hemi-Straw carrier: application of assisted hatching after thawing.", July 2003 (2003-07), HUMAN REPRODUCTION (OXFORD, ENGLAND) JUL 2003 LNKD- PUBMED:12832379, VOL. 18, NR. 7, PAGE(S) 1504 - 1511, XP002526674, ISSN: 0268-1161 * figure 1 *
- LIEBERMANN J ET AL: "Effect of carrier system on the yield of human oocytes and embryos as assessed by survival and developmental potential after vitrification", 1 January 2002 (2002-01-01), REPRODUCTION, BIOSCIENTIFICA LTD, GB, PAGE(S) 483 - 489, XP002526675, ISSN: 1470-1626 * page 485, column 1, paragraph 1-2 *

## Description

### TECHNICAL FIELD

The present invention relates to the field of tools and methods used for manipulating a sample of developmental cells, such as eggs, oocytes and embryos, in a process of cryopreservation. More particularly, the tool of the invention provides a sample loading portion which is retractable into a protective sleeve, for the sample to be protected during further manipulation and storage.

### BACKGROUND

Cryopreservation of developmental cells like eggs, sperm, oocytes, blastocyst, morulae embryos and other early embryonic cells is a powerful tool in assisted reproductive technology. For example, human egg or oocyte preservation by freezing would be a useful treatment option for women who are at risk of losing their ovarian function because of pelvic diseases, surgery or radio/chemotherapy, and it could also avoid some of the moral and ethical issues posed by embryo freezing. Although several pregnancies and live births have been reported using cryopreservation of human eggs, the process of egg preservation by freezing as a technology is still considered to be at an early stage of development. Improving the survival rates of the developmental cells through the freezing and thawing processes thus continues to be a major concern in the field.

Live offspring have resulted from cryopreserved oocytes in mice using slow freezing, however this method has met limited success in other species. Mammalian oocytes have proven to be more difficult to cryopreserve than cleavage-stage embryos, and the efficiency of cryopreservation of oocytes in most species, except for mice, is still very low.

An important problem limiting cryopreservation of oocytes is the low survival rate after the freezing and thawing. Pregnancies and live births after cryopreservation of human oocytes using vitrification were reported, indicating that vitrification is a promising technique.

Vitrification is a process in which glass-like solidification occurs without the formation of ice crystals inside the living cells included within a solution during cooling. Increasing the freezing speed can induce vitrification of developmental cells. The freezing speed can be increased by plunging the sample into a liquid adapted for vitrification, such as liquid nitrogen. Most vitrification protocols use cryoprotectants that dehydrate the cells or embryos to further reduce the creation of ice crystals in an effort to increase the viability of the sample.

Another factor which is thought to decrease the viability of cryopreserved developmental cells is the fact that they are very fragile, and may be damaged by manipulation. This is especially pertinent in freezing protocols in which the sample is manipulated and plunged subsequently into several different solutions.

Various tools are known in the art to manipulate samples during steps of cryopreservation processes. Some art propose pipette style tools in which the sample is sucked into a hollow tube which is then plunged into the solution or liquid nitrogen. This is the case in the United States Patent Application published under No. US 2004/0259072. Other art proposes the use of a "loop" style tools in which a closed loop of nylon or metal wire is affixed at the end of a stem, and is used to pick up the sample. The use of such a tool is discussed in international patent application WO 00/21365. Both theses types of tools present features and limitations which are well known in the art.

Another type of tool is disclosed in international patent application WO 02/085110. This tool has a stem to which a flexible strip made of a liquid nitrogen resistant material is connected, for picking up the sample to be vitrified, and plunging the tool with the sample into liquid nitrogen. The strip is affixed to the stem so one can grasp the tool manually, and manipulate it to displace the strip for the various steps of the cryopreservation procedure. The strip is loaded with a sample and for protection, it is subsequently inserted into a thin elongated cap, the base of which is secured to the stem. Inserting the strip into the cap has been found to be a particularly delicate procedure, because contacting the cap with the sample may result in damage to it and in diminishing the survival rates. The insertion procedure resembles inserting a thread into the eye of a needle except for the additional challenge that the strip must not touch the periphery of the cap. Quite often, while attempting this procedure, the strip and the loaded sample are put into contact with the cap periphery, and the sample is damaged. This decreases the viability of the sample vitrified with this tool.

There thus remains a need in the art for a tool in which the loading strip or portion can be placed into a protected position in a manner which would reduce the risks of damaging the sample and require less dexterity to handle. It is thought that such an advancement would result in higher survival rates and thus be highly beneficial to the fields of cryopreservation of developmental cells and assisted reproductive technology.

### SUMMARY

Accordingly, an object of the present concept is to provide a tool for manipulating developmental cells in a process of cryopreservation, which overcomes at least some of the insufficiencies outlined in the prior art.

According to one aspect, the concept provides a tool for manipulating a sample of developmental cells in a process of cryopreservation. The tool has an elongated stem and a sample loading portion at one end of the stem, and is characterized in that it further comprises a sleeve telescopically mounted onto the stem in a manner that the loading portion is retractable into the sleeve with a spacing defined between the loading portion and an internal portion of the sleeve to accommodate the sample, whereby the sample on the sample loading portion is covered by the sleeve when the loading portion is retracted into the sleeve.

According to still another aspect, the concept provides a method of protecting a sample of developmental cells during a process of cryopreservation. The method comprises: loading the sample onto a loading portion of a sample manipulation tool and retracting the loading portion of the tool within a protective sleeve until the sample is positioned in a spacing defined between the loading portion and an internal portion of the sleeve.

In the instant specification, the term "developmental cells" is intended to refer to a reproductive body of an organism that has the capacity to develop into a new individual organism capable of independent existence. The term developmental cells is used in the plural as including the singular, and includes, but is not limited to, sperm, oocytes, embryos, morulae, blastocysts, and other early embryonic cells. This definition was presented in international patent application no. WO 00/21365.

### BRIEF DESCRIPTION OF THE DRAWINGS

These and other features, aspects and advantages of the present invention will become better understood with reference to the following description and accompanying drawings wherein:
Fig. 1 is a perspective view of a tool in accordance with an embodiment of the present invention, the tool is shown in extended position and accompanied by an optional casing.
Fig. 2 is a perspective view of the tool of Fig. 1, wherein the tool is shown in retracted configuration.
Fig. 3 is a perspective view of the tool of Fig. 1 retracted and inserted within the casing.
Fig. 4 is an exploded perspective view of the tool and casing of Fig. 1, with the tool shown disassembled.
Fig. 5 includes Figs 5A, 5B, 5C and 5D, which illustrate perspective views of alternative embodiments of a groove for the tool of Fig. 1.

### DETAILED DESCRIPTION

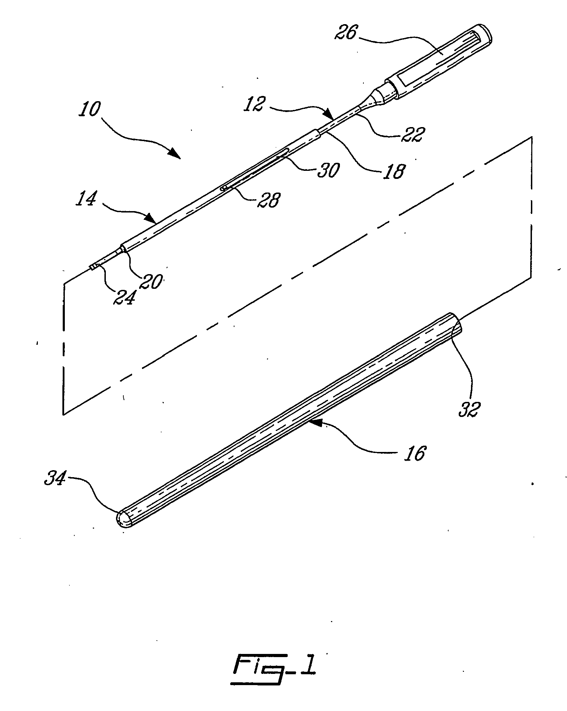

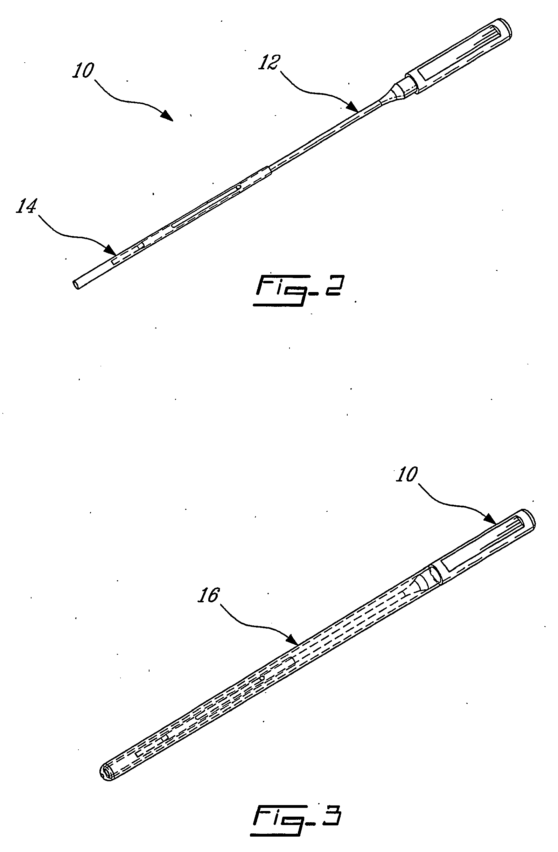

Referring to the drawings, and more specifically to Fig. 1, an embodiment of a tool 10 for manipulating developmental cells in a cryopreservation process is described. The tool 10 includes operating member 12 which is telescopically mounted inside a sleeve 14. The tool 10 is shown accompanied by an optional casing member 16 in which the entire tool may be inserted for protection. In use, the operating member 12 is loaded with a sample of the developmental cells, which is subsequently retracted within the sleeve 14 for protection, as it is depicted in Fig. 2. Then, the entire tool 10 in retracted configuration is inserted within the casing 16 as it is depicted in Fig. 3. One skilled in the art will understand the various possibilities of applications of a tool along the lines of the present invention, in light of the following description. However, the embodiment depicted in the figures is particularly adapted for use in human assisted reproductive technology, for manipulating human oocytes in a vitrification process which shall be described further.

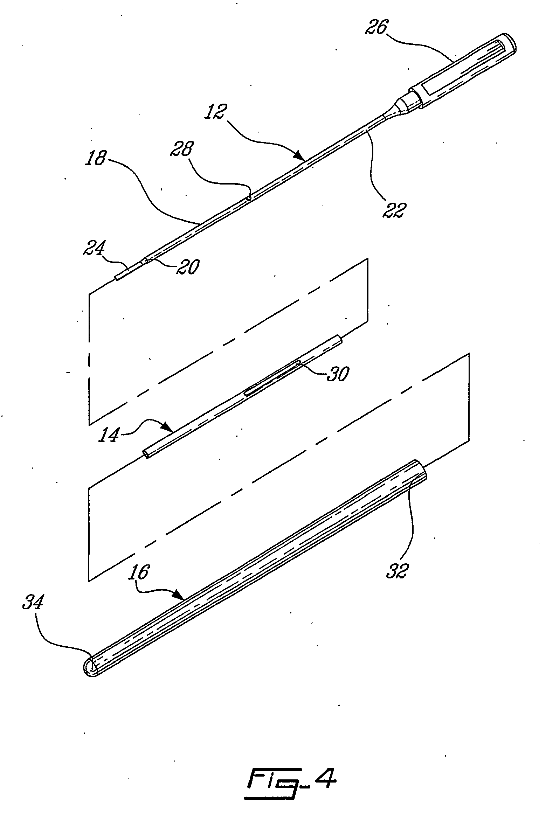

Turning now to Fig.4, the operating member 12 and the sleeve 14 of the tool 10 (Fig. 1) are shown disassembled. The operating member 12 includes an elongated stem 18, which is preferably cylindrical, and which has two opposed ends. The distal end 20 will be referred to as the loading end 20, whereas the proximal end 22 will be referred to as the manipulation end 22. The loading end 20 is connected to a loading portion 24 where the sample of developmental cells is loaded, whereas the manipulation end 22 is preferably connected to a cap 26 which is adapted to engage the casing 16. For illustrative purposes, the stem 18 of this preferred embodiment measures approximately 80 mm in length, and has a diameter of 1.5 mm, whereas the sleeve 14 measures approximately 60 mm in length.

The developmental cells are loaded onto a strip 24 which acts as the loading portion. The strip 24 is connected to the loading end 20 of stem 18 and extends approximately 8 mm outwardly from the stem 18, in the axial direction. The strip 24 is preferably elongated, flat, and flexible. The strip 24 has a generally rectangular shape with a preferred width of approximately 1 mm, and it is made quite thin so as to fit under the lens of a microscope. In addition to being flexible; it should also be transparent for easier manipulation of samples. The preferred thickness of the strip 24 is 0.15 mm.

The sleeve 14 is a cylindrical member having two open ends, thus resembling a straw. Turning back to Figs 1 and 2, it is seen that the operating member 12 is inserted within the sleeve 14 for operation. The stem 18 and the sleeve 14 thus have matching external and internal diameters, respectively, to allow a telescopic movement to take place. The sleeve 14 acts as a protective member for the retracted loading portion 24. In the preferred embodiment, the external diameter of the stem 18 is slightly smaller than the internal diameter of the sleeve 14 to create a sufficient gap between internal and external diameters of the sleeve14 and stem 18, respectively, to enable the stem 18 to slide or telescope freely in a lengthwise movement within the sleeve14. The preferred internal diameter of sleeve 14 is 2 mm, whereas the preferred outer diameter of the stem 18 is of 1.5 mm.

The sleeve 14 is preferably made shorter than the stem 18, to allow the stem 18 to be manipulated from end 22 (or at the optional cap 26) when the loading end 20 arrives flush with the corresponding end of the sleeve 14. By pulling and pushing the manipulation end 22 of the operating member 20 from one end of the sleeve 14, a user may retract and extend the loading portion 24 into and out of the other end of the sleeve 14, respectively.

In order to keep the operating member 12 from being displaced undesirably, or under its own weight, means to limit the telescopic movement are provided. A protuberance 28 and groove 30 (Fig. 1) may be used, or at least a portion of the stem 18 can be made to cooperate with at least an internal portion of the sleeve 14 to create friction. In fact, the telescopic movement of the operating member 12 needs not exceed the displacement necessary to completely retract and extend the loading portion 24 into and out from the sleeve 14. The tool 10 is preferably provided with a groove 30 longitudinally defined in the sleeve 14, and with a protuberance 28 defined on the stem 18 and arranged to mate with the groove 30. Preferably, the protuberance 28 closely matches the size of the groove 30. Once the operating member 12 is assembled to sleeve 14 (see Figs 1 and 2), the protuberance 28 is fitted into groove 30, thus confining it and cooperating therewith to restrict the axial displacement of the operating member 12 to the size and shape of groove 30. The cooperating interaction between the protuberance 28 and groove 30 thus limit the sliding or telescopic movement of the operating member 12 relative to the sleeve 14 within a predetermined range.

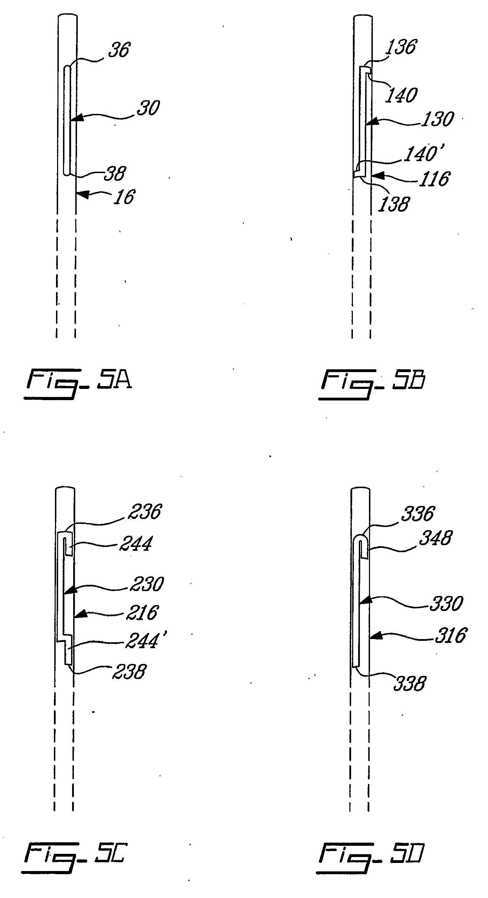

The length of groove 30 defines the total displacement ability of the operating member 12 relative to the sleeve 14, and the length of the groove 30 should thus be at least as long as the strip 24 to allow it to be extended and retracted completely. The relative longitudinal position of the protuberance 28 and of the groove 30 determine the extended position and retracted position of the strip 24. Fig. 5A shows that groove 30 includes a extension end 38 and a retraction end 36. If the groove 30 is only slightly longer than the strip 24, the position of the protuberance 28 and of the groove 30 is chosen such that the strip 24 be completely extended when protuberance 28 abuts the extension end 38 of the groove, and the strip 24 be completely retracted when the protuberance 28 abuts the retraction end 36 of the groove 30. In alternative configurations, the positioning of the protuberance 28 abutting the extension end 38 should correspond to the strip 24 being at least partly extended out of the sleeve 14, to allow loading the sample; and the positioning of the protuberance 28 abutting the retraction end 36 should correspond to the strip 24 being at least completely retracted within the sleeve 14, to keep the sample from potential damage when retracted.

Figs 5B, 5C and 5D illustrate alternatives to the groove of Fig. 5A. Fig. 5B illustrates an alternative groove 130, which comprises radial notches 140 and 140' to block telescopic movement of operating member 12 into a predetermined position relative to the sleeve 14 when protuberance 28 is placed therein by rotating the stem 18. The notches 140 and 140' are disposed at opposite ends 136 and 138 of the groove 130, respectively. The notch 140 is particularly useful in keeping the retracted operating member (Fig. 2) from falling through under its own weight when positioned in an upright position, thus potentially preventing damage to the loaded sample. Radial notches 140 and 140' can be referred to as "simple notches", or simply as "notches".

The groove 230 depicted in Fig. 5C is preferred. Groove 230 comprises an inverted L shaped locking notch 244 at the retraction end 236, and an inverted L shaped locking notch 244' at the extension end 238. Locking notches 244 and 244' secure the telescopic movement of operating member 12 in predetermined positions by necessitating an axial in addition to a radial displacement of operating member 12 for protuberance 28 to be displaced from retraction end 236, to extension end 238 and vice versa. This allows a user to secure the strip 24 (Fig 4) into a desired extended position where the strip is exposed for loading the sample, and to securely protect the strip 24 into a desired retracted position during storage. The locking notches 244 and 244' include the radial component of the simple notches 144 and 144', but additionally include a longitudinal component. The embodiment depicted in Fig. 5D illustrates an alternative groove 330 where only one locking notch 348 is used instead of two, and being located at the retracted end 336 of the groove 330. This locking notch 348 is curvilinear instead of square shaped and has the shape of an inverted J.

In the preferred embodiment, the groove 230 shown in Fig. 5C, is used, even though this groove was not depicted in Figs 1 to 4 for reasons of clarity. Groove 230 has a width of 0.8 mm. The corresponding protuberance 28 is 0.6 mm wide, and extends 0.5 mm radially from the stem 18. The protuberance 28 is disposed approximately at midway between the ends 20, 22, of said stem 18.

Turning back to Fig. 1, it is seen that the tool 10 is preferably provided in combination with a casing 16. The casing 16 is hollow, and has an open end 32, and a closing end 34. It is preferably of the same cross-sectional shape as that of the tool 10, and thus cylindrical. The tool 10 can be inserted within the casing 16, for further protection (see Fig. 3). A cap 26 is used to close the open end 32 of the casing 16 when the tool is inserted therein. Preferably, the cap 26 is provided as part of the operating member 12, and is connected to the loading end 22 of stem 18. This is preferred since it allows a user to both insert the tool 10 into the casing 16 and close the casing 16 in a single step, and provides for easy retrieval of the tool 10 from the casing.

Preferably, the operating member 12 is molded as one piece with the stem 18, the strip 24 the protuberance 28 and the cap 26. If the stem is molded, the entrance of the mold can be designed to form the protuberance 28 during the molding operation. One skilled in the art will understand that the components 24, 28, 26 may be provided separately from the stem 18, and affixed or otherwise connected to the stem 18 by any suitable means. Molding the operating member 12 in one piece has been found to reduce production costs and potentially enhance the commercial profitability of the product. The sleeve 14 and casing 16 may also be molded and each component of the tool can be made of the same material.

Many appropriate materials for constructing the tool will no doubt appear to those skilled in the art. It has been determined that using a material that is slightly flexible, and somewhat transparent is advantageous in that the strip 24, if molded in the same operation, will be more flexible and transparent than the other components due to its thinness. Flexibility of the operating member 12 and the sleeve 14 also provides for inserting the protuberance 28 on the stem portion 18 within the groove 230 when assembling the operating member 12 to the sleeve 14. This is particularly helpful if the gap between the part of the stem 18 including protuberance 28 and the sleeve 14 is made tight to keep the protuberance 28 from freely exiting groove 30 when the tool is assembled. Furthermore, the preferred material preferably has a low expansion coefficient, to prevent the tool components from fissuring or cracking due to the rapid and large temperature change between the room temperature and the freezing temperature. The material must further be resistant to the freezing process and somewhat impervious. It should be liquid-nitrogen resistant. Polypropylene has been found to be a suitable material for making the tool 10. Additives, fillers and reinforcements may be added to polypropylene to achieve desired characteristics. The preferred material used in making the tool is Pro-fax^{™} PD626-H12 resin from Basell polyolefins.

One preferred mode of operation is as follows : In order to pick a sample up with the tool 10, manipulation end 22 is manipulated to fully extend the strip 24 from the sleeve 14, to the configuration illustrated in Fig. 1. The user can lock the strip 24 into the extended loading or "exposure" configuration (Fig. 1) by manipulating the stem 18 so that the protuberance 28 is in the inverted "L" shape 244' (Fig. 5) using the manipulation end 22 of the stem 18 (or the cap 26) while retaining the sleeve 14. The user loads a sample by approaching the strip 24 to the sample, which might be in a medium such as a solution, and on a slide in a microscope. Once the sample, including some of the medium in which it is contained, is loaded onto strip 24, the user retracts the loaded strip 24 into the sleeve 14 for the sample to be protected by the sleeve 14. To do this, the manipulation end 22 is pulled, twisted and pulled by the user to bring the protuberance 28 out of the inverted "L" shape 244'. The strip 24 can be locked in the retracted protection position (Fig. 2) by twisting and pushing the manipulation end 22 so that the associated protuberance 28 is driven into the inverted J shape slot 244 (Fig. 5). When the sample is to be removed from storage, protuberance 28 is taken out of the inverted J shape slot 244, and the loaded strip 24 is extended from the sleeve 14 back into the exposure configuration.

In an exemplary vitrification protocol, human oocytes are first suspended in an equilibration solution at room temperature for 3 to 5 minutes. They are then transferred to a vitrification solution at room temperature for 45-60 seconds and immediately plunged into liquid nitrogen. Once in liquid nitrogen, the strip 24 with the loaded oocyte is retracted into the sleeve 14 as described above, and still while keeping the loaded oocyte in the liquid nitrogen, the tool 10 in retracted position is inserted within the casing 16 for storage as described above (for at least one week). It can be seen here that having the casing cap 26 formed as part of the tool 10 is advantageous. After the desired storage period, the tool 10 is removed from the casing 16, the strip 24 is projected from the sleeve 14, and pulled out of the liquid nitrogen. The oocyte is thereafter directly inserted into a thawing solution for one minute at 37°C. The warmed oocytes are then transferred to a diluent solution for 3 minutes and washed twice in a washing solution for 5 minutes. For exemplary solution compositions, see "High Survival Rate of Bovine Oocytes Matured In Vitro Following Vitrification" by Ri-Cheng CHIAN et al., Journal of Reproduction and Development, Vol. 50, No. 6, 2004. Table 1 below shows results of the vitrification and thawing procedure using a tool in accordance with the invention.

**Table 1. Survival rates of human oocytes cultured in vitro following vitrification and thawing using the tool.**

| Stage of oocytes | No. of oocytes examined | No. of oocytes survived (%) * |
|---|---|---|
| Germinal vesicle | 32 | 32 (100) |
| Metaphase-I | 30 | 30 (100) |
| Metaphase-II | 19 | 19 (100) |
| Total | 81 | 81 (100) |

| | | |
|---|---|---|
| * Identified by morphology. | | |

Subsequent experimentation to those which yielded the results presented in Table 1, using the tool of the invention, have resulted in human pregnancy outcome. These results are briefly exposed in Table 2, below.

**Table 2. Details of embryology profile resulting from subsequent experimentation using the tool.**

| | | No. of oocytes | Remark (%) |
|---|---|---|---|
| | | | |
| Oocytes retrieved | | 6 | |
| | *Metaphase-II* | 2 | 2 oocytes matured at collection |
| | *Metaphase-I* | 2 | 2 oocytes matured at collection |
| | *GV oocytes* | 2 | 2 oocytes matured at collection |
| Oocytes survived | | 4 | (66.7) |
| | *Metaphase-II* | 0 | 0 oocyte survived post-thawing |
| | *Metaphase-I* | 2 | 2 oocytes survived post-thawing |
| | *GV oocytes* | 2 | 2 oocytes survived post-thawing |
| | | | |
| Oocytes fertilized | | 3 | (75.0) |
| | *Metaphase-I* | 1 | 1 oocyte fertilized post-ICSI |
| | *GV oocytes* | 2 | 2 oocytes fertilized post-ICSI |
| | | | |
| Oocytes cleaved | | 3 | (100.0) |
| | *Metaphase-I* | 1 | 1 fertilized oocyte cleaved |
| | *GV oocytes* | 2 | 2 fertilized oocytes cleaved |
| | | | |
| Embryos transferred | | 3 | |
| | *Metaphase-I* | 1 | 1 cleaved 2-cell stage embryo |
| | *GV oocytes* | 2 | 1 cleaved 2-cell stage embryo and |
| | | | 1 cleaved 3-cell stage embryos |
| | | | |
| Outcome | | | |
| | *Serum β-hCG test* | | positive (274.0 U/L 2 wks post-transfer) |
| | *Clinical pregnancy* | 2 | 2 gestation sacs with fetal heartbeats |
| | | | (6 wks post transfer) |

The tool in accordance with the invention was also used in the cryopreservation of other types of developmental cells. For example, the survival results from vitrification and thawing of bovine embryos are presented at Tables 3 and 4, below.

**Table 3. The numbers of apoptotic cells in the blastocysts (Day 8) following vitrification and warming using the tool.**

| Group | No. of blastocysts examined | Total cell numbers / blastocyst | Dead cell numbers / blastocyst |
|---|---|---|---|
| Control | 35 | 106.9 ± 28.1 | 5.0 ± 2.9 |
| Vitrification | 27 | 111.2 ± 25.4 | 9.5 ± 4.0 |

| | | | |
|---|---|---|---|
| * There is no difference between groups. | | | |

**Table 4. Survival and hatching rates of vitrified bovine blastocysts following warming and culture.**

| Group | No. of blastocysts examined | No. of blastocysts survived (Day 7) | No. of blastocysts hatched * (Day 9) |
|---|---|---|---|
| Control | 96 | 96 (100.0) | 60 (62.5) |
| Vitrification | 94 | 94 (100.0) | 58 (61.7) |

| | | | |
|---|---|---|---|
| * There is no difference between groups. | | | |

The dimensions given herein with reference to the tool were given for illustrative purposes, but the sizes and proportions were found to be appropriate for ease of manipulation, specifically in view of manipulating human oocytes, and could be slightly varied while maintaining the general functionality of the tool 10 in view of such an application. For example, the strip 24 could be about 1 mm wide and 0.15 mm thick, but could be slightly longer or smaller than 8 mm. Both the width of the strip 24 and the width of the stem 18 could be varied while maintaining the strip 24 thinner than the stem 18. The length of the sleeve 14 could be varied, for example between 40 and 120 mm, but the stem 18 should remain between 15 and 40 mm longer than the sleeve for ease of manipulation at the manipulation end 22 and to keep the tool 10 from being overly cumbersome during prolonged storage. Other types of developmental cells may be better manipulated by taking certain modifications to the embodiment presented. For example, to accommodate the dimensions of other types of developmental cells, the dimensions may be varied appropriately, the strip 24 interchanged with another type of loading portion, or the tool be presented in an otherwise different configuration.

The description hereby disclosed is meant to be illustrative of an appreciated mode of realization of the invention at the time of the application. Many alternatives and modifications can be made to the illustrative example herein disclosed while remaining within the scope of the invention. The scope of the invention is therefore intended to be determined solely by contemplation of the appended claims.

## Claims

1. A tool for manipulating a sample of developmental cells in a process of cryopreservation, the tool having an elongated stem and a sample loading portion at one end of the stem, the tool being **characterized in that** it further comprises a sleeve telescopically mounted onto the stem in a manner that the loading portion is retractable into the sleeve with a spacing defined between the loading portion and an internal portion of the sleeve to accommodate the sample, whereby the sample on the sample loading portion is covered by the sleeve when the loading portion is retracted into the sleeve.

2. The tool as defined in claim 1 wherein the loading portion is a strip.

3. The tool as defined in claim 2 wherein the strip is transparent.

4. The tool as defined in claim 2 or 3 wherein the strip is elongated, flat, and flexible.

5. The tool as defined in any one of claims 1 to 4 wherein the stem has a protuberance extending axially therefrom, and the sleeve has a longitudinal groove of a width adapted for the protuberance to be inserted into the groove and to be confined therewithin, the extent of the telescopic movement of the sleeve thus being limited by the cooperating protuberance and groove.

6. The tool as defined in claim 5 wherein the groove defines a radial notch at a predetermined longitudinal position along the groove, such that placing the protuberance within the radial notch by rotating the stem prevents the telescopic movement and therefore locks the loading portion into position relative to the sleeve.

7. The tool as defined in claim 5 or 6 wherein said groove extends at least as long as the length of said loading portion, and defines opposed retraction and extension ends; and the positioning of the protuberance into abutment with the extension end of the groove corresponds to the associated loading portion being positioned at least partly extended from the sleeve, and the positioning of the protuberance into abutment with the retraction end of the groove corresponds to the loading portion being at least completely retracted within the sleeve.

8. The tool as defined in claim 7 wherein said groove defines an inverted J shape at the retraction end thereof, allowing engagement of the protuberance at the end of the inverted J shape following a rotation of the stem and a longitudinal displacement thereof, respectively; the J shape groove providing the locking of the loading portion in said at least completely retracted position within the sleeve so as to remain in that position during storage.

9. The tool as defined in claim 7 or 8 wherein said groove defines an inverted L shape at the extension end thereof, allowing engagement of the protuberance at the end of the inverted L shape following a rotation of the stem and a longitudinal displacement thereof, respectively; the L shaped groove allowing the loading portion to be locked in said at least partially extended position from the sleeve for it to remain in that position during loading of the sample.

10. The tool as defined in any one of claims 5 to 9 wherein the stem defines an outer diameter slightly smaller than an inner diameter defined by the sleeve, thus allowing the stem to telescope freely with respect to the sleeve.

11. The tool as defined in any one of claims 1 to 4 wherein at least a portion of said stem cooperates with at least a portion of an internal passage defined inside said sleeve to provide friction therebetween.

12. The tool as defined in any one of claims 1 to 11 in combination with a casing in which the tool can be inserted, wherein a cap for the casing is provided at an end of the stem opposite to the loading portion, thus allowing insertion of the tool in the casing and closing of the casing to be carried out in a single step.

13. The tool as defined in any one of claims 1 to 12 wherein said the stem is molded in one piece with the loading portion.

14. The tool as defined in any one of claims 1 to 13 wherein the tool is made of a material that is liquid-nitrogen resistant and has a low thermal expansion coefficient.

15. The tool as defined in claim 14 wherein the material is polypropylene.

16. The tool as defined in any one of claims 1 to 15 wherein said loading portion includes a flat strip that has a width of about 1 mm, a thickness of about 0.15 mm, and a length of about 8 mm, thus being an appropriate size for manipulation of a sample of oocytes; said sleeve has an inner diameter of about 2 mm and said stem has an outer diameter of about 1.5 mm, thus allowing the sleeve to telescope freely; said sleeve is between 40 and 120 mm long, and said stem is between 15 and 40 mm longer than said sleeve.

17. A method of protecting a sample of developmental cells during a process of cryopreservation, the method comprising: loading the sample onto a loading portion of a sample manipulation tool; and retracting the loading portion of the tool within a protective sleeve until the sample is positioned in a spacing defined between the loading portion and an internal portion of the sleeve.

18. The method as defined in claim 17, further comprising:
inserting the loading portion with the loaded sample into a liquid adapted for vitrifying the sample; and
wherein the step of retracting is done while maintaining said sample within said liquid.

19. The method as defined in claim 18 further comprising:
inserting said tool into a casing while maintaining at least said loaded sample within the liquid; and
capping said container at said step of inserting.

## Patentansprüche

1. Werkzeug zur Bearbeitung einer Probe von Entwicklungszellen in einem Prozess einer Tiefkühlkonservierung, wobei das Werkzeug einen langgestreckten Schaft und einen Probeladungsabschnitt an einem Ende des Schafts aufweist, wobei das Werkzeug **dadurch gekennzeichnet ist, dass** es ferner eine Hülse umfasst, die teleskopartig auf dem Schaft in der Weise befestigt ist, dass der Ladungsabschnitt in die Hülse mit einem Abstand einschiebbar ist, der zwischen dem Ladungsabschnitt und einem inneren Abschnitt der Hülse definiert ist, um die Probe aufzunehmen, wobei die Probe auf dem Probeladungsabschnitt durch die Hülse bedeckt ist, wenn der Ladungsabschnitt in die Hülse eingeschoben ist.

2. Werkzeug nach Anspruch 1, wobei der Ladungsabschnitt ein Streifen ist.

3. Werkzeug nach Anspruch 2, wobei der Streifen durchlässig ist.

4. Werkzeug nach Anspruch 2 oder 3, wobei der Streifen langgestreckt, flach und flexibel ist.

5. Werkzeug nach einem der Ansprüche 1 bis 4, wobei der Schaft einen Vorsprung aufweist, der sich axial von ihm erstreckt, und wobei die Hülse einen längsseitigen Einschnitt mit einer Breite aufweist, die dafür ausgelegt ist, dass der Vorsprung in den Einschnitt eingefügt und darin eingeschlossen werden kann, weshalb das Ausmaß der Teleskopbewegung der Hülse durch das Zusammenwirken des Vorsprungs und des Einschnitts begrenzt wird.

6. Werkzeug nach Anspruch 5, wobei der Einschnitt eine radiale Kerbe an einer vorgegebenen längsseitigen Position entlang des Einschnitts definiert, derart, dass ein Anordnen des Vorsprungs innerhalb der radialen Kerbe die Teleskopbewegung durch eine Drehung des Schafts verhindert und daher den Ladungsabschnitt in der Position relativ zu der Hülse verriegelt.

7. Werkzeug nach Anspruch 5 oder 6, wobei sich der Einschnitt mindestens so weit wie die Länge des Ladungsabschnitts erstreckt und wobei er gegenüberliegende Einziehungs- und Ausdehnungsenden definiert; und wobei das Positionieren des Vorsprungs in Anschlag an dem Ausdehnungsende des Einschnitts dem damit verbundenen Ladungsabschnitt korrespondiert, der dann mindestens teilweise von der Hülse ausgedehnt positioniert ist, und wobei das Positionieren des Vorsprungs in Anschlag an dem Einziehungs-ende des Einschnitts dem Ladungsabschnitt korrespondiert, der dann mindestens vollständig innerhalb der Hülse eingezogen ist.

8. Werkzeug nach Anspruch 7, wobei der Einschnitt eine umgekehrte J-Form an seinem Einziehungsende definiert, was einen Eingriff des Vorsprungs an dem Ende der umgekehrten J-Form ermöglicht, der auf eine Drehung des Schafts bzw. auf eine längsseitige Verschiebung folgt; wobei der J-förmige Einschnitt die Verriegelung des Ladungsabschnitts in der mindestens vollständig eingezogenen Position innerhalb der Hülse bereitstellt, um während der Lagerung in dieser Position zu verbleiben.

9. Werkzeug nach Anspruch 7 oder 8, wobei der Einschnitt eine umgekehrte L-Form an dem Ausdehnungsende desselben definiert, was einen Eingriff des Vorsprungs an dem Ende der umgekehrten L-Form ermöglicht, der auf eine Drehung des Schafts bzw. auf seine längsseitige Verschiebung folgt; wobei der L-förmige Einschnitt die Verriegelung des Ladungsabschnitts in der mindestens teilweise vollständig von der Hülse ausgedehnten Position ermöglicht, um während des Ladens der Probe in dieser Position zu verbleiben.

10. Werkzeug nach einem der Ansprüche 5 bis 9, wobei der Schaft einen äußeren Durchmesse definiert, der kleiner als ein innerer Durchmesser der Hülse ist, womit es dem Schaft daher möglich ist, sich in Bezug auf die Hülse teleskopartig frei ineinanderzuschieben.

11. Werkzeug nach einem der Ansprüche 1 bis 4, wobei mindestens ein Abschnitt des Schafts mit mindestens einem Abschnitt eines inneren Durchgangs, der innerhalb der Hülse definiert ist, zusammenwirkt, um dazwischen eine Reibung bereitzustellen.

12. Werkzeug nach einem der Ansprüche 1 bis 11 in Kombination mit einem Gehäuse, in das das Werkzeug eingesetzt werden kann, wobei eine Kappe für das Gehäuse an einem Ende des Schafts gegenüber dem Ladungsabschnitt vorgesehen ist, womit es daher möglich ist, dass ein Einsatz des Werkzeugs in das Gehäuse und ein Schließen des Gehäuses in einem einzigen Schritt durchgeführt werden können.

13. Werkzeug nach einem der Ansprüche 1 bis 12, wobei der Schaft einteilig mit dem Ladungsabschnitt geformt ist.

14. Werkzeug nach einem der Ansprüche 1 bis 13, wobei das Werkzeug aus einem Material hergestellt ist, das gegenüber flüssigem Stickstoff resistent ist und einen niedrigen thermischen Ausdehnungskoeffizienten aufweist.

15. Werkzeug nach Anspruch 14, wobei das Material Polypropylen ist.

16. Werkzeug nach einem der Ansprüche 1 bis 15, wobei der Ladungsabschnitt einen flachen Streifen enthält, der eine Breite von etwa 1 mm, eine Dicke von etwa 0,15 mm und eine Länge von etwa 8 mm aufweist, womit er eine geeignete Größe für die Bearbeitung einer Probe von Oocyten aufweist; wobei die Hülse einen Innendurchmesser von etwa 2 mm und einen Außendurchmesser von etwa 1,5 mm aufweist, womit es der Hülse möglich ist, sich teleskopartig frei ineinanderzuschieben; wobei die Hülse eine Länge von 40 bis 120 mm aufweist und der Schaft um 15 bis 40 mm länger als die Hülse ist.

17. Verfahren zum Schutz einer Probe von Entwicklungszellen während eines Prozesses einer Tiefkühlkonservierung, wobei das Verfahren Folgendes umfasst: Laden der Probe auf einen Ladungsabschnitt eines Probenbearbeitungswerkzeugs; und Einziehen des Ladungsabschnitts des Werkzeugs in eine Schutzhülse, bis die Probe in einem Abstand positioniert ist, der zwischen dem Ladungsabschnitt und einem inneren Abschnitt der Hülse definiert ist.

18. Verfahren nach Anspruch 17, das ferner Folgendes umfasst:
Einsetzen des Ladungsabschnitts mit der geladenen Probe in eine Flüssigkeit, die dafür ausgelegt ist, die Probe zu verglasen; und
wobei der Schritt des Einziehens getätigt wird, während sich die Probe in der Flüssigkeit befindet.

19. Verfahren nach Anspruch 18, das ferner Folgendes umfasst:
Einsetzen des Werkzeugs in ein Gehäuse, während mindestens die geladene Probe innerhalb der Flüssigkeit gehalten wird; und
Abdecken des Behälters in diesem Schritt des Einsetzens.

## Revendications

1. Outil permettant de manipuler un échantillon de cellules de développement dans un procédé de cryoconservation, l'outil ayant une tige allongée et une portion de chargement d'échantillon à une extrémité de la tige, l'outil étant **caractérisé en ce qu'**il comprend en outre un manchon monté de manière télescopique sur la tige de telle sorte que la portion de chargement puisse être rétractée dans le manchon avec un espacement défini entre la portion de chargement et une portion interne du manchon de manière à recevoir l'échantillon, l'échantillon sur la portion de chargement d'échantillon étant couvert par le manchon lorsque la portion de chargement est rétractée dans le manchon.

2. Outil selon la revendication 1, dans lequel la portion de chargement est une bande.

3. Outil selon la revendication 2, dans lequel la bande est transparente.

4. Outil selon la revendication 2 ou 3, dans lequel la bande est allongée, plate et flexible.

5. Outil selon l'une quelconque des revendications 1 à 4, dans lequel la tige a une protubérance s'étendant axialement depuis celle-ci, et le manchon a une gorge longitudinale ayant une largeur adaptée à la protubérance devant être insérée dans la gorge et devant être confinée à l'intérieur de celle-ci, l'étendue du mouvement télescopique du manchon étant ainsi limitée par la coopération de la protubérance et de la gorge.

6. Outil selon la revendication 5, dans lequel la gorge définit une encoche radiale au niveau d'une position longitudinale prédéterminée le long de la gorge, de telle sorte que le positionnement de la protubérance à l'intérieur de l'encoche radiale par rotation de la tige empêche le mouvement télescopique et par conséquent verrouille la portion de chargement en position par rapport au manchon.

7. Outil selon la revendication 5 ou 6, dans lequel ladite gorge s'étend au moins sur la longueur de ladite portion de chargement, et définit des extrémités opposées de rétraction et d'extension ; et le positionnement de la protubérance en butée avec l'extrémité d'extension de la gorge correspond à la portion de chargement associée positionnée au moins de manière en partie étendue hors du manchon, et le positionnement de la protubérance en butée avec l'extrémité de rétraction de la gorge correspond à la portion de chargement au moins complètement rétractée à l'intérieur du manchon.

8. Outil selon la revendication 7, dans lequel ladite gorge définit une forme en J inversé au niveau de son extrémité de rétraction, ce qui permet l'engagement de la protubérance au niveau de l'extrémité de la forme en J inversé à la suite d'une rotation de la tige et d'un déplacement longitudinal de celle-ci, respectivement ; la gorge en forme de J assurant le verrouillage de la portion de chargement dans ladite position au moins complètement rétractée à l'intérieur du manchon de manière à rester dans cette position au cours du stockage.

9. Outil selon la revendication 7 ou 8, dans lequel ladite gorge définit une forme en L inversé au niveau de son extrémité d'extension, ce qui permet l'engagement de la protubérance au niveau de l'extrémité de la forme en L inversé à la suite d'une rotation de la tige et d'un déplacement longitudinal de celle-ci, respectivement ; la gorge en forme de L permettant à la portion de chargement d'être verrouillée dans ladite position au moins en partie étendue hors du manchon de manière à ce qu'elle reste dans cette position au cours du chargement de l'échantillon.

10. Outil selon l'une quelconque des revendications 5 à 9, dans lequel la tige définit un diamètre extérieur légèrement inférieur à un diamètre intérieur défini par le manchon, ce qui permet le télescopage libre de la tige par rapport au manchon.

11. Outil selon l'une quelconque des revendications 1 à 4, dans lequel au moins une portion de ladite tige coopère avec au moins une portion d'un passage interne défini à l'intérieur dudit manchon de manière à fournir une friction entre elles.

12. Outil selon l'une quelconque des revendications 1 à 11, en combinaison avec un boîtier dans lequel l'outil peut être inséré, un capuchon pour le boîtier étant prévu au niveau d'une extrémité de la tige opposée à la portion de chargement, ce qui permet d'effectuer en une seule étape l'insertion de l'outil dans le boîtier et la fermeture du boîtier.

13. Outil selon l'une quelconque des revendications 1 à 12, dans lequel ladite tige est moulée d'une seule pièce avec la portion de chargement.

14. Outil selon l'une quelconque des revendications 1 à 13, dans lequel l'outil est fabriqué en un matériau résistant à l'azote liquide et présentant un faible coefficient de dilatation thermique.

15. Outil selon la revendication 14, dans lequel le matériau est du polypropylène.

16. Outil selon l'une quelconque des revendications 1 à 15, dans lequel ladite portion de chargement comporte une bande plate présentant une largeur d'environ 1 mm, une épaisseur d'environ 0,15 mm et une longueur d'environ 8 mm, ce qui est une taille appropriée pour la manipulation d'un échantillon d'ovocytes ; ledit manchon a un diamètre intérieur d'environ 2 mm et ladite tige a un diamètre extérieur d'environ 1,5 mm, ce qui permet le télescopage libre du manchon ; ledit manchon mesure entre 40 et 120 mm de long, ladite tige est entre 15 et 40 mm plus longue que ledit manchon.

17. Procédé pour protéger un échantillon de cellules de développement au cours d'un processus de cryoconservation, le procédé comprenant : le chargement de l'échantillon sur une portion de chargement d'un outil de manipulation d'échantillon ; et la rétraction de la portion de chargement de l'outil à l'intérieur d'un manchon de protection jusqu'à ce que l'échantillon soit positionné dans un espacement défini entre la portion de chargement et une portion interne du manchon.

18. Procédé selon la revendication 17, comprenant en outre :
l'insertion de la portion de chargement avec l'échantillon chargé dans un liquide prévu pour la vitrification de l'échantillon ; et
l'étape de rétraction étant effectuée tout en maintenant ledit échantillon à l'intérieur dudit liquide.

19. Procédé selon la revendication 18, comprenant en outre :
l'insertion dudit outil dans un boîtier tout en maintenant au moins ledit échantillon chargé à l'intérieur du liquide ; et
le recouvrement dudit récipient lors de ladite étape d'insertion.
